# EUROPEAN PATENT SPECIFICATION

(11) **EP 0 830 855 B1**
(45) Date of publication and mention of the grant of the patent: **03.12.2003**
(21) Application number: 96306860.6
(22) Date of filing: 20.09.1996
(51) Int. Cl.: A61F 5/448

(54) **An ostomy coupling**
Ostomiekupplung
Accouplement stomique

(43) Date of publication of application: 25.03.1998
(73) Proprietor: BRISTOL-MYERS SQUIBB COMPANY, Princeton, NJ 08543-4000 (US)
(72) Inventor: Steer, Peter Leslie, East Grinstead, Sussex RH10 4JZ (US)
(74) Representative: Holmes, Miles Keeton

(56) References cited:
- EP-A- 0 286 501
- GB-A- 2 244 652
- GB-A- 2 289 221
- US-A- 4 203 432
- US-A- 5 322 522

## Description

This invention relates to an ostomy coupling.

The term "ostomy" is intended to include colostomy, ileostomy, urostomy and other surgical diversion procedures.

Ostomy couplings are used to connect and disconnect a bag for receiving a stomal discharge to and from a medical grade adhesive pad which is applied to the peristomal area of the skin of the wearer. Many designs of ostomy coupling are known. One which has enjoyed considerable world-wide commercial success is described and claimed in U.K. Patent No. 1,571,657.

An ostomy coupling in which locking and unlocking is achieved by deforming a ring is disclosed in our U.K. Patent Application No. 2,289,221A published on 15 November 1995.

An ostomy appliance in which a V-section ring (see U.S. Patent 5,322,522) holds coupling members together is disclosed in U.S. Patent No. 5,322,523 and in European Patent 572 378B. Features of the design of U.S. Patent No. 5,322,523 are that inwardly sprung tongues on the ring peripherally surround the joined coupling parts and that a press-button engagement device as well as a hook and detent engagement device are included, apparently in a quest for secure location of the locking ring on the coupling parts. It appears inevitable that quite intricate manipulation of this design of coupling is needed when applying or removing the bag.

It has been proposed by Kubo, in Japanese Utility Model No. 62-11610, published February 1985, that an ostomy device should have a double female ring structure which can interengage with a male ring. The male ring may be on the bag and the female ring on a skin-attachable adhesive pad, or vice-versa. An outer ring on the female coupling is circular and flexible and has a pair of inwardly-extending catches at opposite ends of a diameter. By pressing on two diametrically extending lugs, whose diameter is substantially at right angles to the diameter joining the catches, the outer female ring is deformed so that the catches are caused to move radially outwardly, so permitting separation of the two coupling parts. This arrangement, though perhaps operable in theory, has serious disadvantages in practice. To the best of the present Applicant's knowledge and belief, it has not come into medical use anywhere in the world. A further disadvantage of Kubo and of many present day ostomy couplings is that they extend outwardly from the body an undesirable distance, and so cause bulges or bumps under the wearer's clothing.

An ostomy coupling designed by the present Inventor is described in UK patent application No. 96 07520.5 (published as GB-A-2299761). This comprises two coupling parts and a springy split ring carrying locking tabs. These tabs can enter holes in one of the coupling parts to hold the parts together, and can be withdrawn by rotating the ring to allow the parts to be separated.

Reference is also made to GB 2 289 221 which describes an ostomy coupling having a first coupling element and a second coupling element, the former being of substantially channel shape in cross-section and the second element being of complementary closed loop form and having a rib extending upwardly from a flange. The rib carries a peripheral deflectable seal. The two coupling elements are connectible by a deformable ring which is made captive to one coupling element at one circumferential region thereof and which has a member constrained to move in a radial direction which, when so moved, causes a deformation of the ring whereby tabs thereon are shifted clear of overlap with a rim portion of the other coupling element, so permitting separation of the two coupling elements.

In one aspect, the invention provides an ostomy coupling comprising a first coupling member and a second coupling member which are mutually engageable and surround a region which includes a stomal orifice, the coupling also including a resilient ring which encircles the two coupling members,
the ring comprising first and second limbs extending on opposite sides of a handle, the handle being manipulable to cause the ring to be deformed such that radially inwardly extending tabs on the limbs are shifted between respective first positions in which the ring is undeformed and the tabs lock the two coupling members together, and respective second positions in which the ring is deformed in such a way as to shift the tabs radially outwardly to positions where they permit separation of the two coupling members;
wherein the ring is arranged to deform in the same manner as a split ring such that ends of the limbs remote from the handle move away from each other when the ring is deformed; characterised in that:
the ring further comprises a deformable joining portion integral or non integral with the limbs, the deformable joining portion being elastic and extending between said ends of the limbs for permitting said ends of the limbs to move away from each other when the ring is deformed, and for urging the limbs to return towards each other.

In a second aspect, the invention provides an ostomy coupling comprising a first coupling member and a second coupling member which are mutually engageable and surround a region which includes a stomal orifice, the coupling also including a resilient ring which encircles the two coupling members,
the ring comprising first and second limb portions extending on opposite sides of a handle, the handle being manipulable to cause the ring to be deformed such that radially inwardly extending tabs on the limb portions are shifted between respective first positions in which the ring is undeformed and the tabs lock the two coupling members together, and respective second positions in which the ring is deformed in such a way as to shift the tabs radially outwardly to positions where they permit separation of the two coupling members;
wherein the ring is arranged to deform in the same manner as a split ring such that ends of the limb portions remote from the handle move away from each other when the ring is deformed; characterised in that:
the ring further comprises a deformable joining portion integral with the limb portions and joining said ends of the limb portions, the deformable portion permitting said ends of the limb portions to move away from each other when the ring is deformed.

According to a preferred embodiment of the invention, the limbs of the ring are made of a relatively rigid but springy plastics material, for example an acetal resin, and the tabs carried by it are provided with curved or angled ramp surfaces which, when the ring is deformed by a force tending to rotate it relative to the coupling members, causes the tabs to be withdrawn from slots so permitting the two coupling members to be separated.

In an advantageous embodiment of the invention, two springy limbs of the endless ring each carry a pair of inwardly projecting tabs, the ring is rotatable relative to one of the coupling members, and rotary movement of the ring may be limited by suitably-positioned arcuately-spaced studs on the first coupling member.

A valuable feature of the invention is that the ring is reliably maintained on the first coupling member even when the second coupling member is removed therefrom. Additionally, when the first and second coupling members are coupled together, the handle on the ring is accessible at an upper region of the coupling, and can be shifted between its two limit positions. In one of these the coupling is locked and in the other, unlocked. The manipulation needed to shift the ring in a rotary direction is simple and straightforward, and can be readily achieved even by the elderly or infirm. The ring because of its springy nature immediately springs back to its "handle top dead centre" position once the wearer of the coupling releases the handle.

In one preferred embodiment of the invention, the endless ring has two limbs which together extend around about 350° and a thread of resilient plastics material joining the ends of these limbs. This thread material is sufficiently strongly resilient that it exerts a force on the ends of the limbs sufficient to prevent them moving apart in their normal rest position. In that position, the coupling is held closed by engagement of tabs in holes. This force can be overcome by rotating the ring relative to the body side coupling member.

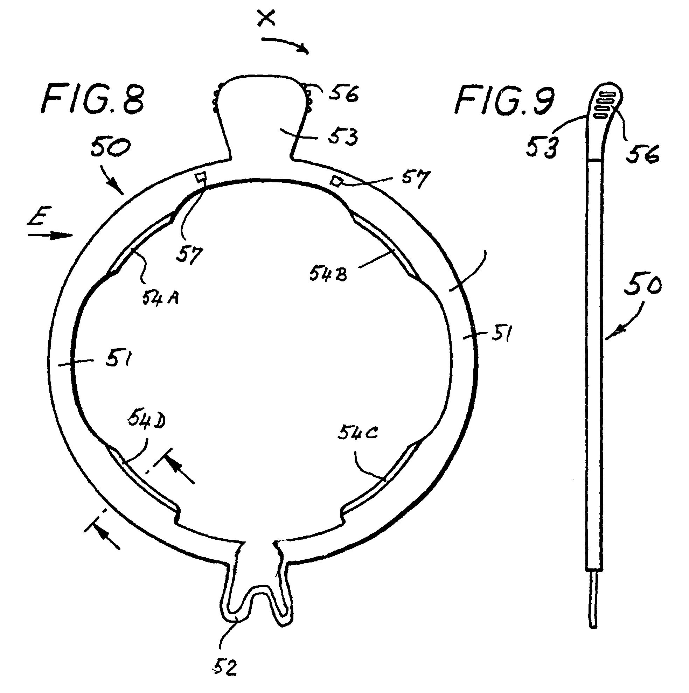

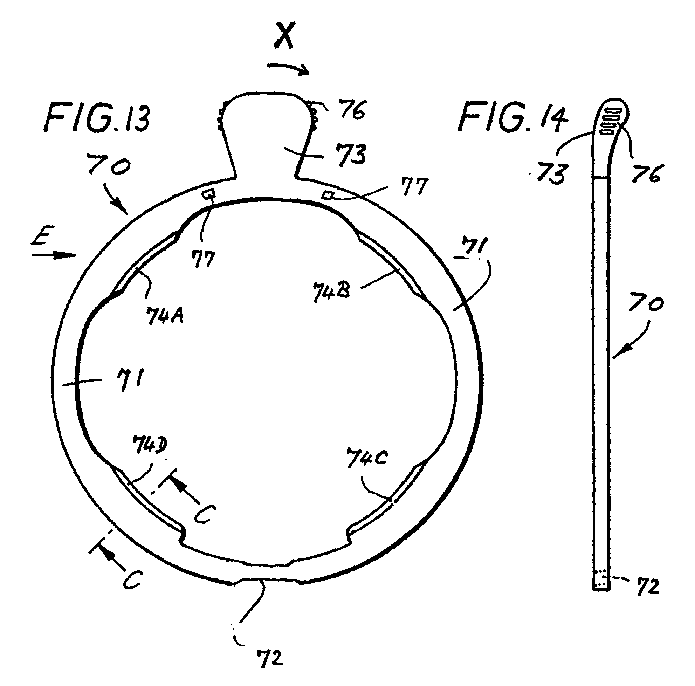

In a second preferred embodiment of the invention, the endless ring has two limbs which are joined by a short length of elastic plastics material. This material likewise exerts a force tending to hold the ring closed, although of course this force can be overcome by a manual rotational shifting of the ring, achieved by a movement of the handle as indicated by arrow X of Figure 8 or 13.

In another embodiment of the invention, the means for joining the ends of two limbs of the ring may consist of a commonplace elastic band, each of the opposed ends of the respective limbs then being provided with a suitable hook so that the elastic band can be temporarily attached between the limb ends. Other suitable joining arrangements may occur to one skilled in the art.

The invention will be better understood from the following description of an illustrative example thereof, given with reference to the accompanying drawings in which:-
Figures 1-4 illustrate a first coupling member which in the realisation of the invention as an ostomy coupling is a body side coupling member, Figure 1 being a front elevation, Figure 2 a partial cross-section in an axial plane, Figure 3 a cross-section on the plane B-B in Figure 2, and Figure 4 a view similar to Figure 2 but on a larger scale and showing only one end of a diameter;
Figures 5, 6 and 7 show a plan view, a partial axial section, and a sectional view on an enlarged scale of a second coupling member which, in the realisation of the invention as an ostomy coupling, would be a bag side coupling member having an ostomy pouch or bag secured to a surface of its flange;
Figure 8 shows one embodiment of ring usable in a coupling according to this invention;
Figure 9 is a view of the ring of Figure 8, looking in the direction E (Fig. 8);
Figures 10-12 are cross-sectional views showing the principle of operation of the disclosed coupling; these illustrate in a cross-sectional plane containing the axis of rotation of a coupling, the mutual engagement and locking of two coupling members, one end of a diameter only being shown; and
Figures 13 and 14 illustrate one alternative form of locking ring, and Figures 15 and 16 illustrate a further design of locking ring, which is currently preferred for certain applications.

The present invention has been developed with a view to meeting the need for an ostomy coupling which can be easily manipulated by old or infirm and sometimes confused persons to separate a bag side coupling from a body side coupling member which is attached to a medical grade adhesive pad. As well as permitting easy manipulation, the invention aims to substantially avoid pressure on the tender peristomal area and to provide clear and unmistakable indications when the coupling members are (a) unlocked and ready for separation, and (b) locked together. This is a feature of importance to ostomates, who need to feel confident that no leakage will occur, and that inadvertent detachment is virtually impossible. Another valuable feature of the invention is that, upon the wearer releasing the handle, the ring springs back to its rest position, which may be "top dead centre" but could be a different position. This "automatic" return of the lock ring to its normal position is believed to be most useful in practice and to be novel in ostomy couplings. The wearer does not need to touch the handle when fitting a bag-side coupling to the body side coupling in place on the body; but to remove it the handle is rotated either way to an "unlock" position. Hence one can only get the bag off when the handle is held in the "unlock" positions and once the bag is off the lock automatically springs back when released, thereby ensuring automatic locking upon the fitting of the next bag.

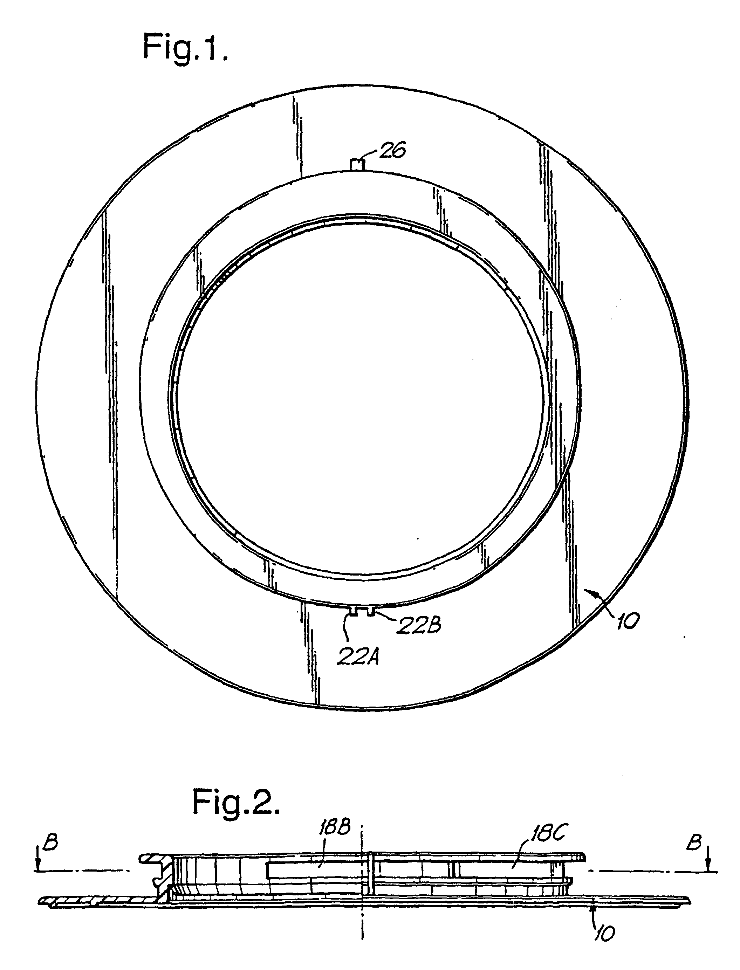

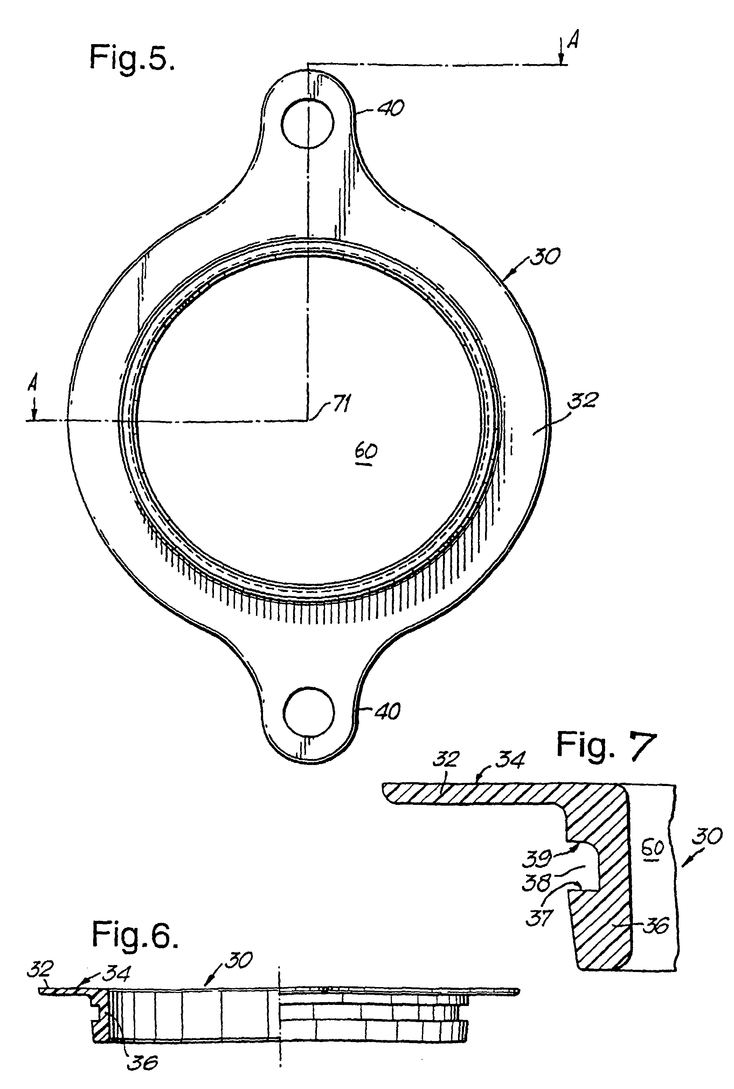

A preferred embodiment of a coupling according to the present invention comprises three parts, a first coupling member 10, Figures 1-4; a second coupling member 30, Figures 5 to 7; and three alternative versions 50, 70, 80 of a locking ring, Figures 8 and 9, Figures 13 and 14, and Figures 15 and 16.

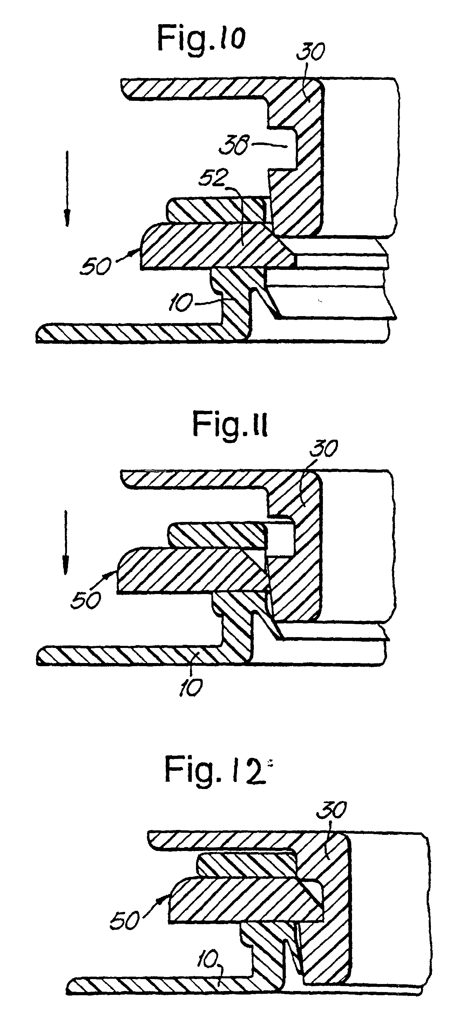

Referring firstly to Figures 10 to 12, the bag side coupling member 30 in use is presented to and engaged with the body side coupling member 10 which is attached to the peristomal area in the normal way. The member 30 is pushed into the body side member 10 as shown by the arrow. Figure 9 shows an intermediate stage in the coupling operation; the ring 50 has been deformed so that a limb thereof is shifted radially outwardly as the part 30 slides past the chamfer on the tab 52 of the ring 50. Figure 10 shows the completion of the action; ring 50 springs back into the recess 38 in the part 30 and holds the two coupling members locked together. Uncoupling is effected by a rotational movement of the ring 50 achieved by pushing the handle portion of the ring to rotate the ring, as will be explained in more detail later in this specification.

Referring now to Figures 1-4, the first coupling member 10 will usually be the body side coupling member, and the second, the bag side member. However, without departing from the invention, the first coupling member could be the bag side member and the second coupling member could be the body side member, although this arrangement is currently less preferred.

The body side coupling member 10 may be an injection moulding made of high or low density polyethylene, or EVA, and comprises a flange 12 having a surface 14 which in practice is attached in any suitable way such as by adhesive or by heat or RF or ultrasonic welding to a pad of medical grade adhesive. The purpose of such a medical grade adhesive pad is to attach the ostomy appliance to the skin of the wearer. The pad comprises a base which is preferably a thin film of polymeric material such as polyethylene and an adhesive layer situated on the rear surface of a base. Such an adhesive layer is preferably formed as a homogeneous blend of one or more pressure-sensitive viscous or elastomeric materials having intermittently dispersed therein one or more water-soluble or swellable hydrocolloid gums and may also include one or more thermoplastic elastomers and/or one or more swellable cohesive strengthening agents. Medical grade adhesive pads of other compositions may alternatively be used.

The body side coupling member 10 has a peripheral wall 16, which as seen in Figure 2 is circular with apertures 18A, 18B and 18C therein. As shown, there are three apertures but in a presently-preferred design of body side coupling, there are four apertures, each receiving one of the four tabs illustrated in Figure 8 or Figure 13. The aperture 18A at the top of the coupling member (in normal position of wear) subtends about 65°, and each of the apertures 18B and 18C subtends about 50°. In one embodiment in which there are four apertures, they would be located at 90° centres, and each aperture would subtend about 30°, although apertures of other extent or other position could be used. While in a preferred embodiment of the invention and as illustrated the first and second coupling members are generally circular in form, the invention is not considered to be limited to this, and the coupling members instead could be oval or other closed-loop shape. For brevity of description, however, circular coupling members are referred to. The wall 16 is upstanding from the flange 12. A further flange 17 extends radially outwardly from the wall 16.

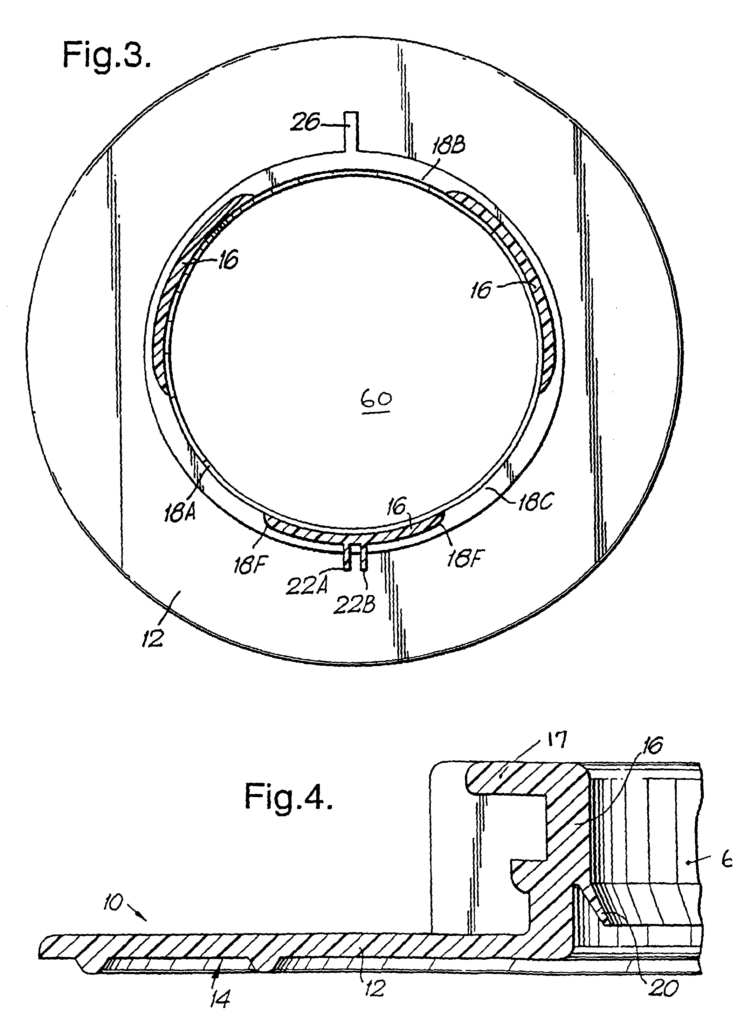

At the base of the wall 16, extending inwardly, is a continuous, optionally resilient, deflectable sealing strip 20 (Fig. 4) which extends around the stomal orifice 60. The chief function of this strip is to inhibit leakage but it also serves to accommodate tolerances if during moulding of the coupling members, some slight divergence from the designed dimensions should occur.

At the opposite end of a diameter from the aperture 18B, on the external surface of the wall 16, there is provided a stop which is formed by a pair of nibs or walls 22A,22B, which project outwardly from the wall 16 and are also integral with the flange 14. Of course a single rib could be used instead. The end of the wall 16 remote from the flange 12 may optionally have an external chamfer. As seen in Figure 10, in the assembled condition of the coupling members, the wall 16 of the body side member 10 surrounds the bag side member 30.

A lug 26, Figure 1 or 3, extends radially outwardly externally of the wall 16, and serves to limit the extent of arcuate motion of a handle portion 51 of a locking ring, as will be later described.

The flange 17 of the body side coupling member 10 is shaped so that its maximum width is at a region which is at the top of the coupling in the normal position of wear. Its width decreases as one moves around the coupling to the 90° and 270° locations; its width is uniform over substantially the bottom half of the coupling. The purpose of this widening of the flange is to more securely retain the ring on the body side coupling member.

Referring now to Figures 5, 6 and 7, these depict a bag side coupling member 30 which is of simple design. The bag side coupling member 30 may also be an injection moulding and may be of EVA. It comprises a flange 32, having a surface 34 to which an ostomy bag or pouch (not shown) is attached. Extending from the flange, at right angles thereto (although other angles would be possible) is a cylindrical wall 36 which, as indicated above, in use fits within the wall 16 of the first coupling member. A groove 38 in the radially external surface of the wall 36 extends completely around that wall and has a flat surface 37 remote from the flange 32 and a curved surface 39 nearer to the flange 32, these surfaces 37 and 39 bounding the channel or groove 38. Belt attachment tabs 40 and a pull tab (not shown) may optionally be included. However, the inventor's present view is that such tabs can be dispensed with since, in the unlocked condition, the two coupling members can be quite readily separated by simply grasping the flange 32 between finger and thumb and pulling the second coupling member directly (axially) away from the first coupling member 10. As will be appreciated, the first and second coupling members surround a stomal orifice 60, into which normally projects the stoma of the person who has undergone surgery.

Referring now to Figures 8 and 9, these depict one preferred embodiment of locking ring 50 for use in the present invention. This is a ring comprising two springy limbs 51 and a resilient springy joining portion and is of generally circular formation. The limbs 51 and the joining portion 52 made of a relatively rigid but springy plastics material such as an acetal resin. For example, good results have been achieved with an acetal copolymer known as "KEMATAL" (Regd. Trade Mark) which is also referred to as polyoxymethylene (POM) and is available from Hoechst. This is a crystalline thermoplastic with an exceptionally stable polymer structure; a suitable grade is "HOSTAFORM" (Regd. Trade Mark) C.27021.

The ring 50 is depicted in Figure 8 in its normal or unstressed condition. It comprises a handle 53 and four inwardly projecting tabs, 54A, 54B, 54C and 54D. The tabs are located around the ring at about 90 degree spacing, as shown. The tabs 54 project inwardly from an inner surface of the ring 50 and are integral with it. The extent of inward projection is determined according to the radius of the ring. As will be appreciated, ostomy couplings are made in various sizes, ranging from about 32 millimetres to about 57 millimetres or more. The larger sizes would be used post-operatively, and would have tabs which are wider in the radial direction.

The tabs 54A and 54B are provided with smoothly-sloping end portions 54E. The tabs 54C and 54D have smoothly sloping surfaces 54F. The smooth shallow slope of the surfaces 54E and 54F assist in enabling the handle (and hence the ring 50) to be freely movable within limits in a rotational direction.

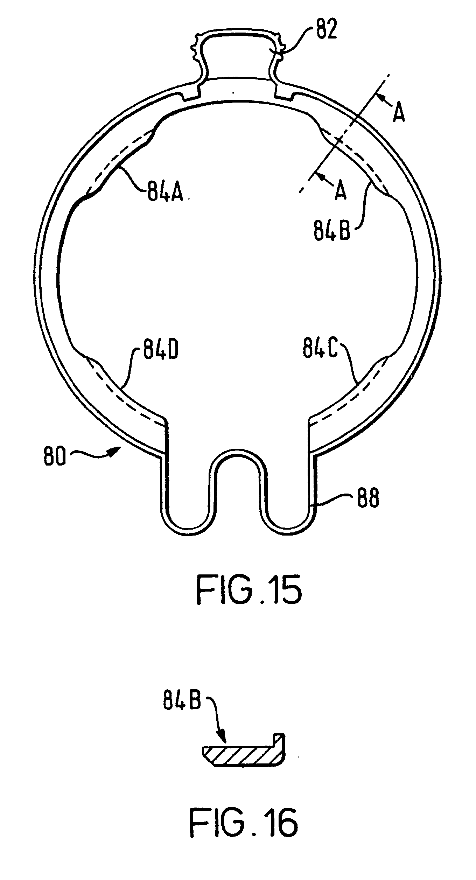

The handle 51 optionally has ribs 56 thereon (see Figs. 8 and 13) to provide a rough surface which can be gripped. This makes shifting the ring an easier task for elderly or infirm users. To avoid the handle 51 catching in clothing, it is preferably canted inwards, towards the body side coupling member, by a few degrees, as seen in Figure 9 or 14. The ring 50 carries a pair of stops 57, which limit the rotation of the ring in either rotary direction. One or other of the limit stops 57 comes into contact with the lug 26 (Fig.1), when the ring 50 is rotated relative to the body side coupling 10.

When it is desired to unlock the coupling and thereby release the bag side member so that it may be axially drawn off the body side coupling member, the handle 51 is rotated a short distance, e.g. up to about 15°, in either rotary direction away from its central position.

As a result, the tabs 54 are shifted circumferentially in one or other rotary direction, and a surface of each tab rides up on the edge of the corresponding aperture 18 seen in Figure 2. Thus, the tabs are forced in directions approximately radially outwardly, this being permitted by the springy nature of the material of the ring 50.

As a consequence of rotation of the handle, the tabs 54 as stated are forced generally radially outwardly and clear the groove 38 of the bag-side coupling. Also the sinuous joining portion 52 is somewhat straightened, while continuing to hold the ring on the bodyside coupling, due to its inherent resilience. With the ring shifted rotationally relative to the coupling part 10, it is readily seen that the tabs are withdrawn from the stomal orifice 60 and also withdrawn from the channel 38. The bag and bag side coupling member thereon can be removed from the body side coupling member by a gentle axial pull. Referring now to Figures 13 and 14, these show an alternative design of locking ring 70 which however has many resemblances to the locking ring 50. In Figures 13 and 14, like parts to those in Figures 8 and 9 are given corresponding reference numerals but in the 70s rather than the 50s. The significant difference between ring 50 and ring 70 is that the ring 70 is made of two limbs 71 of acetal resin (or like springy plastics) and a joining portion 72 of a plastics elastomer whose thickness is substantially equal to the thickness of the limbs 71 but whose width (radial extent) is slightly less than that of the limbs 71. The plastics elastomer chosen is such that it is stretchable to up to about 150 per cent of its length at rest. The joining portion 72 could alternatively be made of rubber or synthetic rubber.

Figures 15 and 16 illustrate a further alternative design of locking ring 80 which is mainly circular and has a handle 82, four tabs 84A, 84B, 84C and 84D, two stop members 86, and a springy joining portion 88 which is of sinuous shape, and may be of approximately one millimeter in thickness. The ring 80 is of a springy nature, and is preferably made of an acetal resin, for example that known as DELRIN 500 - NC10 (acetal) available from Du Pont (UK) Limited, Hemel Hempstead, Hertfordshire. The handle 82 is provided with ribs to enable it to be more securely grasped with finger and thumb. As with the rings shown in Figures 8 and 13, the tabs have curved surfaces. One advantageous aspect of either of the joining portions 52 (Fig.8) or 88 (Fig.15) is that their connection to the ends of the limbs of the ring 50 or 80 results in a force being applied to the ring ends tending to urge the tabs 54C and 54D (or the tabs 84C and 84D) inwardly in a generally radial direction. This assists in ensuring that these tabs remain securely engaged within the groove 38 (Fig.7) even if the spring resilience of the remainder of the ring has diminished somewhat due to use or ageing of the material of the ring. The elastic portion 72 or the "means for joining" referred to above serve a similar purpose. In use rotation of the ring causes the surfaces of the tabs to engage the edge of the relevant aperture and be moved generally radially outwardly so as to permit the coupling parts to be separated from one another. The tab shape can be seen from Figure 16 which is a cross-section on the plane A-A of Figure 15.

The present invention can be employed in a design of ostomy coupling which has a body-side member which includes an annular channel. One such design is shown in European Patent Application No. 96302557.2 (EP-A-0 737 456). The present invention could be employed in this design simply by including a springy or elastic joining portion located between and joined to the ends 23, 24 of the split ring 20 shown in Figure 3 of the said European Patent Application. Such a modification would increase the security provided by the ring 20.

It will be seen that the particular embodiments of the invention described herein provides couplings which can be easily disassembled and assembled by persons who are not nimble, which can be assembled and disassembled with only very light pressure being applied either during coupling or uncoupling to the tender peristomal area, and which can readily be made in any coupling size. The rings 50, 70 or 80 are in effect made captive within the coupling members 10 and 30, and so cannot escape; moreover, the positive rotational movement of the handle between two fixed positions gives assurance to the wearer such that he or she knows when the coupling is properly locked, because in the properly locked condition, the handle is at top dead centre. If desired, by choosing an appropriate springiness of the material for the locking ring, the ring can be made to automatically spring back towards top dead centre position when the wearer releases his/her grip of the handle. While a ring having four tabs is currently preferred, three or five or any other convenient number could be employed.

## Claims

1. An ostomy coupling comprising a first coupling member (10) and a second coupling member (30) which are mutually engageable and surround a region which includes a stomal orifice, the coupling also including a resilient ring (50; 70; 80) which encircles the two coupling members,
the ring comprising first and second limbs (51, 71) extending on opposite sides of a handle (53; 73; 82), the handle being manipulable to cause the ring to be deformed such that radially inwardly extending tabs (54A-D; 74A-D; 84A-D) on the limbs are shifted between respective first positions in which the ring is undeformed and the tabs lock the two coupling members together, and respective second positions in which the ring is deformed in such a way as to shift the tabs radially outwardly to positions where they permit separation of the two coupling members;
wherein the ring is arranged to deform in the same manner as a split ring such that ends of the limbs remote from the handle move away from each other when the ring is deformed; **characterised in that**
the ring further comprises a deformable joining portion (52; 72; 88) integral or non integral with the limbs, the deformable joining portion being elastic and extending between said ends of the limbs for permitting said ends of the limbs to move away from each other when the ring is deformed, and for urging the limbs to return towards each other.

2. An ostomy coupling comprising a first coupling member (10) and a second coupling member (30) which are mutually engageable and surround a region which includes a stomal orifice, the coupling also including a resilient ring (50; 70; 80) which encircles the two coupling members,
the ring comprising first and second limb portions (51, 71) extending on opposite sides of a handle (53; 73; 82), the handle being manipulable to cause the ring to be deformed such that radially inwardly extending tabs (54A-D; 74A-D; 84A-D) on the limb portions are shifted between respective first positions in which the ring is undeformed and the tabs lock the two coupling members together, and respective second positions in which the ring is deformed in such a way as to shift the tabs radially outwardly to positions where they permit separation of the two coupling members;
wherein the ring is arranged to deform in the same manner as a split ring such that ends of the limb portions remote from the handle move away from each other when the ring is deformed; **characterised in that**
the ring further comprises a deformable joining portion (52; 72; 88) integral with the limb portions and joining said ends of the limb portions, the deformable portion permitting said ends of the limb portions to move away from each other when the ring is deformed.

3. An ostomy coupling according to claim 1 or 2 wherein the deformable joining portion (52; 72; 88) is generally opposite the handle.

4. An ostomy coupling according to claim 2, wherein the deformable portion is elastic and urges said ends of the limbs towards each other.

5. An ostomy coupling according to claim 1 or 2 in which the limbs (51; 71) are made of a relatively rigid but springy plastics material, for example an acetal resin, and the tabs (54A-D; 74A-D; 85A-D) carried by it are provided with curved or angled ramp surfaces which, when the ring is deformed by a force tending to rotate it relative to the coupling members, causes the tabs to be withdrawn so permitting the two coupling members (10, 30) to be separated.

6. An ostomy coupling accordingly to claim 1, 2 or 3 in which the ring is an endless ring carrying inwardly projecting tabs, the ring being rotatable relative to one of the coupling members (10), and rotary movement of the ring being limited by suitably-positioned arcuately-spaced studs (26, 22A, 22B) on the first coupling member.

7. An ostomy coupling according to any of claims 1 to 4 in which when the first and second coupling members are coupled together, the handle (53; 73; 82) on the ring is accessible at an upper region of the coupling, and can be shifted between its two limit positions, whereby in one of these, the coupling is locked and in the other, unlocked.

8. An ostomy coupling according to claim 6 or 7 in which the ring (50; 70; 80), at one limit position of rotation, has its handle (53; 73; 82) at top dead centre.

9. An ostomy coupling according to any preceding claim, wherein the limbs (51) of the ring together extend around about 350°, and the deformable joining portion comprises a thread (52; 88) of resilient plastics material joining the ends of the limbs portions.

10. An ostomy coupling according to claim 9, wherein the thread (52; 88) is of a stretchable elastic thermoplastics material.

11. An ostomy coupling according to claim 9 or 10 in which the thread material (52; 88) is sufficiently strongly resilient that it exerts a force on the ends of the limb portions sufficient to prevent them moving apart in their normal rest position.

12. A locking ring for use in an ostomy coupling, comprising two springy limbs and a joining portion.

13. A locking ring according to claim 8 in which the joining portion is a stretchable elastic thermoplastics material.

14. A locking ring according to claim 8 in which the joining portion is a sinuous springy resilient thread-like element.

15. A locking ring according to any preceding claim in which the joining portion is a thread of springy acetal resin plastics which exerts a force on the limbs preventing them from springing apart.

## Patentansprüche

1. Ostomiekupplung mit einem ersten Kupplungsglied (10) und einem zweiten Kupplungsglied (30), die miteinander koppelbar sind und einen Bereich einschließen, der einen Stomaaustritt umfasst, wobei die Kupplung auch einen Federring (50; 70; 80) enthält, der die beiden Kupplungsglieder umschließt,
wobei der Ring erste und zweite Arme (51; 71) aufweist, die sich auf gegenüberliegenden Seiten eines Handgriffs (53; 73; 82) erstrecken, und der Handgriff betätigbar ist, dass er eine Verformung des Rings derart erzeugen kann, dass auf den Armen radial nach innen aufsitzende Nasen (54A-D; 74A-D; 84A-D) verschoben werden zwischen jeweiligen ersten Positionen, in denen der Ring nicht verformt ist und die Nasen die zwei Kupplungsglieder zusammenschließen, und jeweiligen zweiten Positionen, in denen der Ring so verformt ist, dass die Nasen radial nach außen in Positionen geschoben werden, wo sie ein Trennen der beiden Kupplungsglieder erlauben;
wobei der Ring in gleicher Weise zur Verformung wie ein Spaltring eingerichtet ist, so dass sich die vom Handgriff entfernt liegenden Enden der Arme voneinander weg bewegen, wenn der Ring verformt wird; **dadurch gekennzeichnet, dass**
der Ring außerdem ein verformbares Verbindungsstück (52; 72; 88) aufweist, das fest oder lose mit den Armen verbunden ist, wobei das verformbare Verbindungsstück elastisch ist und sich zwischen die Enden der Arme erstreckt, um zu ermöglichen, dass sich die Enden der Arme voneinander weg bewegen, wenn der Ring verformt wird, und dass die Arme gezwungen werden, wieder zueinander zurückzukehren.

2. Ostomiekupplung mit einem ersten Kopplungsglied (10) und einem zweiten Kupplungsglied (30), die miteinander koppelbar sind und einen Bereich einschließen, der einen Stomaaustritt umfasst, wobei die Kupplung auch einen Federring (50; 70; 80) enthält, der die beiden Kupplungsglieder umschließt,
wobei der Ring erste und zweite Armstücke (51; 71) aufweist, die sich auf gegenüberliegenden Seiten eines Handgriffs (53; 73; 82) erstrecken, und der Handgriff betätigbar ist, dass er eine Verformung des Rings derart erzeugen kann, dass auf den Armstücken radial nach innen aufsitzende Nasen (54A-D; 74A-D; 84A-D)verschoben werden zwischen jeweiligen ersten Positionen, in denen der Ring nicht verformt ist und die Nasen die zwei Kupplungsglieder zusammenschließen, und jeweiligen zweiten Positionen, in denen der Ring so verformt ist, dass die Nasen radial nach außen in Positionen geschoben werden, wo sie eine Trennen der beiden Kupplungsglieder erlauben;
wobei der Ring in gleicher Weise zur Verformung wie ein Spaltring eingerichtet ist, so dass sich die vom Handgriff entfernt liegenden Enden der Armstücke voneinander weg bewegen, wenn der Ring verformt wird; **dadurch gekennzeichnet, dass**
der Ring außerdem ein verformbares Verbindungsstück (52; 72; 88) aufweist, das fest mit den Armstücken verbunden ist und die Enden der Armstücke verbindet, wobei das verformbare Stück es den Enden der Armstücke ermöglicht, sich voneinander weg zu bewegen, wenn der Ring verformt wird.

3. Ostomiekupplung nach Anspruch 1 oder 2, **dadurch gekennzeichnet, dass** das verformbare Verbindungsstück (52; 72; 88)im Allgemeinen dem Handgriff gegenüber liegt.

4. Ostomiekupplung nach Anspruch 2, **dadurch gekennzeichnet, dass** das verformbare Stück elastisch ist und die Armenden aufeinander zu zwingt.

5. Ostomiekupplung nach Anspruch 1 oder 2, in der die Arme (51; 71) aus relativ festem aber federndem Kunststoffmaterial, z.B. einem Acetalharz, bestehen und die auf ihnen aufsitzenden Nasen (54A-D; 74A-D; 85A-D) mit gekrümmten oder winkligen Rampenflächen versehen sind, welches dann, wenn der Ring durch eine Kraft verformt wird, die ihn mit Bezug auf die Kupplungsglieder zu drehen versucht, die Nasen dazu bringt, so zurückzutreten, dass die beiden Kupplungsglieder (10; 30) getrennt werden können.

6. Ostomiekupplung nach Anspruch 1,2 oder 3, in welcher der Ring ein Endlosring ist, auf dem nach innen vorspringende Nasen sitzen, wobei der Ring drehbar mit Bezug auf eines der Kupplungsglieder (10) ist und die Drehbewegung des Ringes durch geeignet auf einem Bogen in Abständen angeordnete Stifte (26, 22A, 22B) auf dem ersten Kupplungsglied begrenzt wird.

7. Ostomiekupplung nach einem der Ansprüche 1 bis 4, in der dann, wenn das erste und zweite Kupplungsglied aneinander gekoppelt sind, der Handgriff (53; 73; 82) auf dem Ring in einem oberen Bereich der Kupplung zugänglich ist und zwischen seinen beiden Grenzlagen verstellt werden kann, wobei in einer von diesen die Kopplung geschlossen und in der anderen nicht geschlossen ist.

8. Ostomiekupplung nach Anspruch 6 oder 7, in welcher der Ring (50; 70; 80) in der einen Grenzlage der Drehung seinen Handgriff (53; 73; 82) im oberen Totpunkt hat.

9. Ostomiekupplung nach einem der vorhergehenden Ansprüche, **dadurch gekennzeichnet, dass** die Arme (51) des Ringes sich zusammen über etwa 350° erstrecken und dass das verformbare Verbindungsstück einen Faden (52; 88) aus federndem Plastmaterial umfasst, der die Enden der Armstücke verbindet.

10. Ostomiekupplung nach Anspruch 9, **dadurch gekennzeichnet, dass** der Faden (52; 88) aus einem dehnbaren elastischen thermoplastischen Material besteht.

11. Ostomiekupplung nach Anspruch 9 oder 10, in der das Fadenmaterial (52; 88) ausreichend stark federnd ist, so dass es eine Kraft auf die Enden der Armstücke ausübt, die groß genug ist, um ihre Bewegung voneinander weg in ihre gewöhnliche Ruhelage zu verhindern.

12. Verschlussring zur Verwendung in einer Ostomiekupplung mit zwei federnden Armen und einem Verbindungsstück.

13. Verschlussring nach Anspruch 12, in dem das Verbindungsstück ein dehnbares elastisches thermoplastisches Material ist.

14. Verschlussring nach Anspruch 12,in dem das Verbindungsstück ein gewundenes federndes elastisches fadenförmiges Element ist.

15. Verschlussring nach Anspruch 12, 13 oder 14 in dem das Verbindungsstück ein Faden aus einem federnden Acetalharzkunststoff besteht, der eine Kraft auf die Arme ausübt, die sie am Auseinanderspringen hindert.

## Revendications

1. Raccord de stomie comprenant un premier élément de raccord (10) et un second élément de raccord (30) qui peuvent être mutuellement mis en prise et qui entourent une région qui comprend un orifice de stomie, le raccord comprenant également un anneau élastique (50 ; 70 ; 80) qui encercle les deux éléments de raccord,
l'anneau comprenant des première et seconde branches (51 ; 71) s'étendant des côtés opposés d'une poignée (53 ; 73 ; 82), la poignée pouvant être manipulée pour amener l'anneau à être déformé de sorte que des pattes s'étendant radialement vers l'intérieur (54A-D ; 74A-D ; 84A-D) sur les branches sont déplacées entre des premières positions respectives dans lesquelles l'anneau n'est pas déformé et les pattes verrouillent ensemble les deux éléments de raccord et des secondes positions respectives dans lesquelles l'anneau est déformé de manière à déplacer les pattes radialement vers l'extérieur vers des positions où elles permettent une séparation des deux éléments de raccord,
dans lequel l'anneau est agencé pour se déformer de la même manière qu'un anneau fendu de telle sorte que les extrémités des branches distantes de la poignée s'écartent l'une de l'autre lorsque l'anneau est déformé, **caractérisé en ce que**
l'anneau comprend en outre une partie de jonction déformable (52 ; 72 ; 88) faisant partie intégrante ou non des branches, la partie de jonction déformable étant élastique et s'étendant entre lesdites extrémités des branches pour permettre auxdites extrémités des branches de s'écarter l'une de l'autre lorsque l'anneau est déformé et destiné à repousser les branches pour qu'elles reviennent l'une vers l'autre.

2. Raccord de stomie comprenant un premier élément de raccord (10) et un second élément de raccord (30) qui peuvent être mutuellement mis en prise et qui entourent une région qui comprend un orifice de stomie, le raccord comprenant également un anneau élastique (50 ; 70 ; 80) qui encercle les deux éléments de raccord,
l'anneau comprenant des première et seconde parties de branches (51 ; 71) s'étendant sur des côtés opposés d'une poignée (53 ; 73 ; 82), la poignée pouvant être manipulée pour amener l'anneau à être déformé de telle sorte que des pattes s'étendant radialement vers l'intérieur (54A-D ; 74A-D ; 84A-D) sur les parties de branche soient déplacées entre des premières positions respectives dans lesquelles l'anneau n'est pas déformé et les pattes verrouillent ensemble les deux éléments de raccord et des secondes positions respectives dans lesquelles l'anneau est déformé de manière à déplacer les pattes radialement vers l'extérieur à des positions où elles permettent une séparation des deux éléments de raccord,
dans lequel l'anneau est agencé pour se déformer de la même manière qu'un anneau fendu de telle sorte que les extrémités des parties de branche distantes de la poignée s'écartent l'une de l'autre lorsque l'anneau est déformé, **caractérisé en ce que**
l'anneau comprend en outre une partie de jonction déformable (52 ; 72 ; 88) faisant partie intégrante des parties de branche et réunissant lesdites extrémités des parties de branche, la partie déformable permettant auxdites extrémités des parties de branche de s'écarter l'une de l'autre lorsque l'anneau est déformé.

3. Raccord de stomie selon la revendication 1 ou 2, dans lequel la partie de jonction déformable (52 ; 72 ; 88) est généralement opposée à la poignée.

4. Raccord de stomie selon la revendication 2, dans lequel la partie déformable est élastique et repousse lesdites extrémités des branches l'une vers l'autre.

5. Raccord de stomie selon la revendication 1 ou 2, dans lequel les branches (51 ; 71) sont faites d'une matière plastique relativement rigide mais faisant ressort, par exemple une résine acétal, et les pattes (54A-D ; 74A-D ; 85A-D) portées par celles-ci sont munies de surfaces inclinées incurvées ou en angle qui, lorsque l'anneau est déformé par une force tendant à le faire tourner par rapport aux éléments de raccord, amène les pattes à être retirées, permettant ainsi aux deux éléments de raccord (10, 30) d'être séparés.

6. Raccord de stomie selon la revendication 1, 2 ou 3, dans lequel l'anneau est un anneau sans fin portant des pattes dépassant vers l'intérieur, l'anneau pouvant tourner par rapport à l'un des éléments de raccord (10) et le mouvement rotatif de l'anneau étant limité par des ergots positionnés de façon appropriée, espacés suivant un arc (26, 22A, 22B) sur le premier élément de raccord.

7. Raccord de stomie selon l'une quelconque des revendications 1 à 4, dans lequel, lorsque les premier et second éléments de raccord sont accouplés, la poignée (53 ; 73 ; 82) sur l'anneau est accessible au niveau d'une région supérieure du raccord et peut être déplacée entre ses deux positions limites, grâce à quoi dans l'une de celles-ci, le raccord est verrouillé, et dans l'autre, déverrouillé.

8. Raccord de stomie selon la revendication 6 ou 7, dans lequel l'anneau (50 ; 70 ; 80), à une position limite de rotation, a sa poignée (53 ; 73 ; 82) au point mort haut.

9. Raccord de stomie selon l'une quelconque des revendications précédentes, dans lequel les branches (51) de l'anneau s'étendent ensemble sur environ 350°, et la partie de jonction déformable comprend un fil (52 ; 88) de matière plastique élastique réunissant les extrémités des parties de branches.

10. Raccord de stomie selon la revendication 9, dans lequel le fil (52 ; 88) est d'une matière thermoplastique élastique étirable.

11. Raccord de stomie selon la revendication 9 ou 10, dans lequel le matériau du fil (52 ; 88) est suffisamment fortement élastique pour qu'il exerce une force sur les extrémités des parties de branches suffisante pour les empêcher de s'écarter dans leur position normale de repos.

12. Anneau de verrouillage destiné à une utilisation dans un raccord de stomie, comprenant deux branches élastiques et une partie de jonction.

13. Anneau de verrouillage selon la revendication 12 dans lequel la partie de jonction est une matière thermoplastique élastique étirable.

14. Anneau de verrouillage selon la revendication 12 dans lequel la partie de jonction est un élément en forme de fil élastique sinueux faisant ressort.

15. Anneau de verrouillage selon la revendication 12, 13 ou 14 , dans lequel la partie de jonction est un fil de matière plastique de résine acétal faisant ressort qui exerce une force sur les branches les empêchant de s'écarter en sautant.
